## Europäisches Patentamt
## European Patent Office
## Office européen des brevets

(19)

(11) Publication number: **0 023 379**
**B1**

(12)

# EUROPEAN PATENT SPECIFICATION

(45) Date of publication of patent specification: **27.07.83**

(21) Application number: **80200725.2**

(22) Date of filing: **25.07.80**

(51) Int. Cl.³: **C 07 C 45/34,**
**C 07 C 45/39,**
**C 07 C 49/403,**
**C 07 C 29/05, C 07 C 35/08**

(54) **Method for the preparation of cyclohexanol and/or cyclohexanone.**

(30) Priority: **26.07.79 NL 7905781**

(43) Date of publication of application:
**04.02.81 Bulletin 81/5**

(45) Publication of the grant of the patent:
**27.07.83 Bulletin 83/30**

(84) Designated Contracting States:
**BE DE FR GB IT NL**

(56) References cited:
**FR - A - 2 273 783**

**DERWENT JAPANESE PATENTS GAZETTE,
Section Ch: Chemical 25/10/1978, Week A 37,
Unexamined section E General Chemistry, page
2, no. E15 abstract no. 65958A/37**
**CHEMICAL ABSTRACTS, vol. 90, no. 23, 4th
June 1979, page 621, column 1, no. 186481u
Columbus, Ohio, U.S.A.**
**CHEMICAL ABSTRACTS, vol. 89, no. 23, 4th
December 1978, page 585, column 2, no.
197051j Columbus, Ohio, U.S.A.**
**PATENTS ABSTRACT OF JAPAN, vol. 3, no. 19,
17th February 1979, page 116C37**

(73) Proprietor: **STAMICARBON B.V.**
**Postbus 10**
**NL-6160 MC Geleen (NL)**

(72) Inventor: **van Geem, Paul Christiaan**
**Constantijn Hugostraat 13**
**NL-6176 BS Spaubeek (NL)**
Inventor: **de Graaf, Theodorus Franciscus Maria**
**Reinierstraat 2**
**NL-6191 SH Beek (L.) (NL)**
Inventor: **Knol, Dirk**
**Oranjesingel 2**
**NL-6191 XX Beek (L.) (NL)**
Inventor: **Plantema, Otto Gerrit**
**Braakpeel 1**
**NL-6034 RP Nederweert Eind (NL)**

(74) Representative: **Hoogstraten, Willem Cornelis
Roeland et al,**
**OCTROOIBUREAU DSM Postbus 9**
**NL-6160 MA Geleen (NL)**

Courier Press, Leamington Spa, England.

# Method for the preparation of cyclohexanol and/or cyclohexanone

The invention relates to a method for the preparation of cyclohexanol and/or cyclohexanone by hydrogenating benzene to cyclohexene, hydrating the cyclohexene to cyclohexanol and/or oxidating it to cyclohexanone, recovering the cyclohexanol and/or cyclohexanone, and returning unconverted benzene and/or cyclohexene to the hydrogenating step.

Such a method is known from the JP—A 53,090,242 laid open to inspection (Derwent abstract 65968 A) for the preparation of cyclohexanol. A serious problem in applying the known method is formed by the cyclohexane formed as byproduct in applying the known methods for the hydrogenation of benzene to cyclohexene, unless an economically unjustified, extremely low degree of benzene conversion is applied. In applying the known method the cyclohexane is separated from the benzene-cyclohexene-cyclohexane mixture which remains after separation of the cyclohexanol from the reaction mixture of the hydration step, in order to be able to return the remaining benzene-cyclohexene mixture to the hydrogenation step. Cyclohexane, however, is very hard to separate from benzene and/or cyclohexene, and recourse must be had to expensive methods such as extractive distillation. In applying the known method it is, therefore, necessary to keep the degree of benzene conversion low in order to allow only a minimum quantity of cyclohexane to be formed, so that an economic process operation can not actually be achieved.

The invention provides a solution for this problem. According to the invention, cyclohexanol and/or cyclohexanone is prepared by hydrogenating benzene to cyclohexene, hydrating the cyclohexene to cyclohexanol and/or oxidating it to cyclohexanone, recovering the cyclohexanol and/or cyclohexanone and returning unconverted benzene and/or cyclohexene to the hydrogenation step, this process being characterized in that the mixture of cyclohexane with benzene and/or cyclohexene, which has also been obtained during the separation of cyclohexanol from the reaction mixture of the hydration step and/or of cyclohexanone from the reaction of the oxidation step, is subjected to a dehydrogenation reaction, and the benzene thus obtained is returned to the hydrogenation step.

The method according to the invention has the great advantage that the difficult separation of cyclohexane from mixtures with benzene and/or cyclohexene can be dispensed with. Losses of expensive extractive distillation agent are avoided. Neither is any cyclohexane left as by-product for which a use will have to be found. In the hydrogenation step a higher degree of benzene conversion can be applied, so that economic process operation has become possible.

The hydrogenation of benzene to cyclohexene can be carried out by every known method. A suitable method has been described, for instance, in the DE—A 2,221,137, which is referred to for the purpose of brevity. According to this known method, the hydrogenation is carried out at a hydrogen pressure of about 10—50,000 kPa, at a temperature of 0—250°C in the presence of water and an alkaline agent, e.g. an aqueous alkali hydroxide solution. As catalyst for the hydrogenation of benzene to cyclohexene, particularly ruthenium, rhodium and palladium compounds are eligible, more specifically ruthenium compounds, or reduction products thereof. Preferably relatively high degrees of benzene conversion are applied, e.g. such as correspond with 25—75 moles % of cyclohexane in the product mixture. The degree of benzene conversion is preferably at least 30% and is most preferably 40—80%.

The hydration of cyclohexene to cyclohexanol can be effected by every known method. Mostly an acid catalyst is used in this process. Very suitable methods are described in the GB—A 1,381,149 and 1,542,996, which are referred to for the purpose of brevity. Sulphuric acid is very suitable as catalyst. As cocatalyst ferrous sulphate can be used. The hydration is mostly carried out as a process with separate steps, viz. 1) addition of the acid to the double bond of the cyclohexene with formation of the ester of cyclohexanol and the acid, e.g. cyclohexyl hydrogen sulphate, and 2) hydrolysis of the cyclohexyl ester to cyclohexanol and the acid. The first step is carried out, e.g., at a temperature of between −50°C and +30°C, though temperatures of 30—100°C are possible as well, and the second step at 50—150°C. The hydration of the cyclohexene can be carried out also with catalysts other than sulphuric acid, e.g. with a strongly acid ion exchanger, e.g. a cross-linked polystyrene resin containing sulphonic acid groups, or phosphoric acid. The hydration of the cyclohexene is effected in the presence of the benzene not converted in the hydrogenation step and the cyclohexane formed there. The oxidation of cyclohexene to cyclohexanone can be effected by every known method. A suitable method for the oxidation of cyclohexene is described in Angewandte Chemie, *71* (5), page 176 and following pages, which is referred to for the purpose of brevity. An acid aqueous solution of $PdCl_2$ is suitable as catalyst. As cocatalyst e.g. $CuCl_2$, $Fe_2(SO_4)_3$, $K_2Cr_2O_7$ and $K_2S_2O_8$ can be used. The oxidation can be carried out as a process with separate steps; in the first step oxidation of cyclohexene to cyclohexanone takes place by reaction of cyclohexene with the catalyst solution in which the catalyst is reduced, in the second step the catalyst, after separating of the organic phase from the reaction mixture, is brought back in the oxidated state by means of an oxygen containing gas (e.g. air) and is recirculated in the first step. The first step can be carried out at a temperature of between 0 and 150°C and a pressure of between 0.05 and 5 MPa. The second step can be carried out at a temperature of between 0 and 250°C and at a pressure of between 0.05 and 200 MPa. The oxidation of the cyclohexene is effected in the presence of the benzene not converted in the hydrogenation step and the cyclohexane formed there.

After separation of the aqueous phase from the reaction mixture of the hydration and/or of the oxidation step, an organic phase remains, which, besides cyclohexanol and/or cyclohexanone, contains benzene, cyclohexane and possibly unconverted cyclohexene. From this mixture cyclohexanol and/or cyclohexanone is separated, e.g. by distillation, in which process, also a fraction of benzene, cyclohexane and, possibly cyclohexene with a boiling point lower than that of cyclohexanol and/or cyclohexanone is obtained.

In applying the method according to the invention it is not necessary to separate the latter fraction with difficulty into its components. On the contrary, it can be fed as a whole to the dehydrogenation step, where cyclohexane is dehydrogenated to benzene. The cyclohexene, which may also be present, is converted, in this step, into benzene as well. The dehydrogenation can, e.g., be carried out in the gas phase, with a catalyst containing an element from group VI or group VIII of the Periodic Table of Elements, e.g. chromium, molybdenum, nickel, palladium or platinum. Suitable catalysts are, e.g. platinum/aluminium oxide, platinum/carbon, molybdic oxide/aluminium oxide, chromic oxide/aluminium oxide and palladium nickel alloy. The reaction temperature is, e.g., 200—650°C, the pressure e.g. 10—1000 kPa. For very suitable realization reference is made, for the purpose of brevity, to the US—A 3,682,838 and 3,752,776.

The benzene obtained is returned to the hydrogenation step, in which procedure the accumulation of undesired byproducts, e.g. methyl cyclopentane can be prevented by the use of a drain. The hydrogen obtained in the dehydrogenation step is also returned to the hydrogenation step.

From the crude cyclohexanol obtained pure cyclohexanol can be obtained by distillation. If desired, the cyclohexanol can be dehydrogenated by a known method to cyclohexanone, which can be used as feedstock for the preparation of caprolactam. From the crude cyclohexanone obtained in the oxidation pure cyclohexanone can be obtained also by distillation.

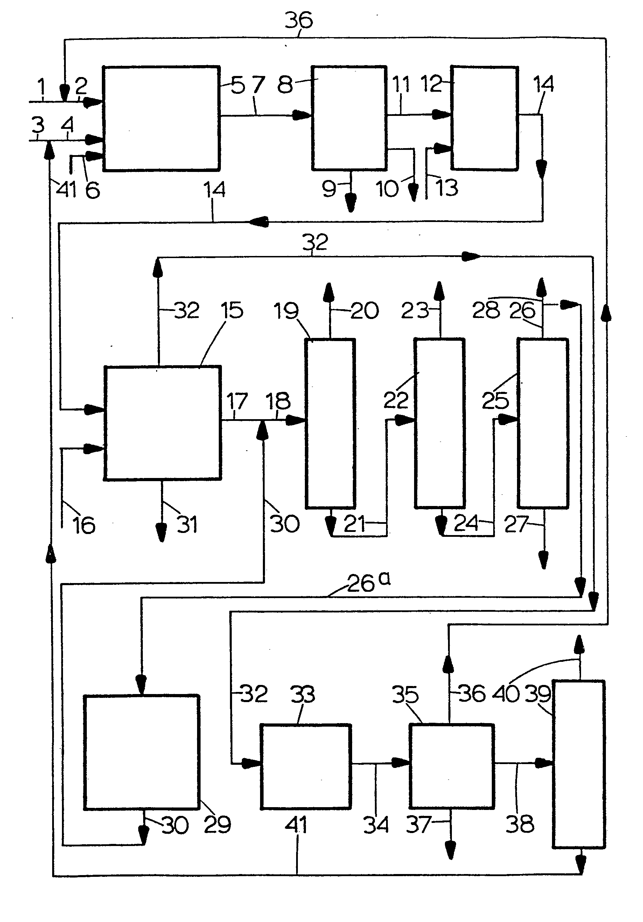

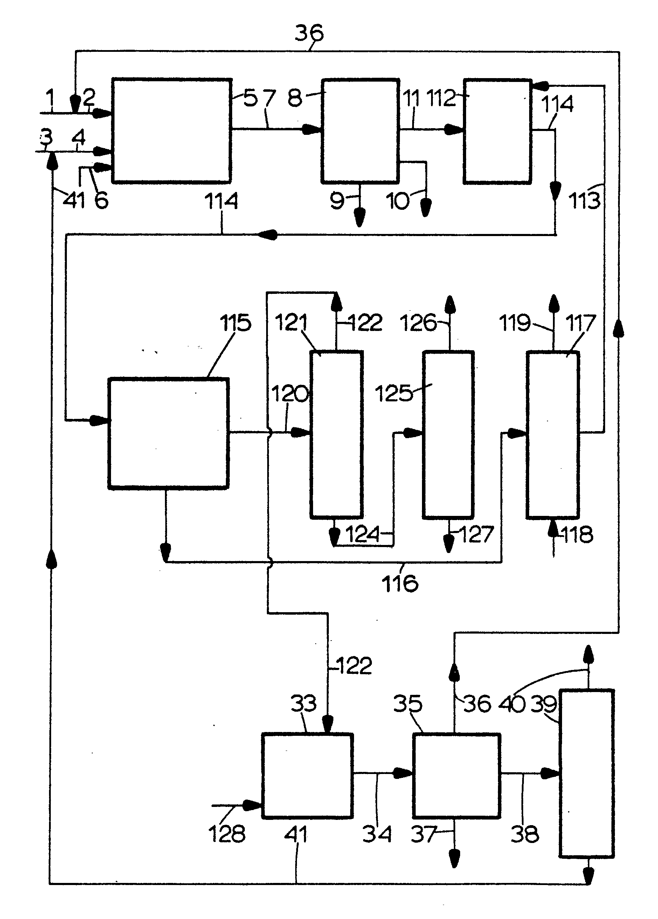

The invention is further elucidated by means of the attached figures. Figure 1 represents a possible flow diagram of an embodiment, figure 2 of another embodiment. In both figures the same numbers mean the same.

Figure 1: Through line 1/2 hydrogen and through line 3/4 benzene is fed to hydrogenation reactor 5. Here the benzene is hydrogenated at a temperature of 200°C and a pressure of about 22 MPa with the aid of a ruthenium-titanium-zinc catalyst supplied through line 6. The catalyst has been obtained by conversion of 1 part by weight of $RuCl_3 \cdot 3 H_2O$ with 1.2 parts by weight of $TiCl_3$, 1 part by weight of $ZnCl_2$ and 65 ml water, followed by 15 ml of 20 N aqueous sodium hydroxide solution. The hydrogenation is carried out in the liquid phase at a hydrogen pressure of 20 MPa. The degree of benzene conversion is 57%, while 53% of the converted benzene is converted into cyclohexene, and the rest almost completely into cyclohexane. Non-converted hydrogen is recirculated (not drawn). The liquid mixture obtained is passed, through line 7, to separator 8, where, for the purpose of recovering the catalyst, it is filtrated and separated into an organic phase and an aqueous phase. The catalyst is recovered through line 9 and the aqueous phase is carried off through line 10. The organic phase is fed, through line 11, to sulphuric-acid addition reactor 12. Through line 13 a 60% by-weight aqueous sulphuric acid solution containing 1% by weight of ferrous sulphate is fed to reactor 12. The addition reaction is effected, with reflux cooling, at 80°C and atmospheric pressure, and at a cyclohexene/sulphuric acid molar ratio of 0.5 to 1. The reaction mixture is subsequently fed, through line 14, to ester hydrolysis reactor 15. Through line 16 so much water is fed that the sulphuric acid concentration is lowered to 30% by weight. The ester hydrolysis is carried out at 110°C and atmospheric pressure. The liquid product mixture is separated in a non-drawn separator into an organic layer consisting of crude cyclohexanol and a layer containing aqueous sulphuric acid, while non-converted cyclohexene, benzene and cyclohexane leave hydrolysis reactor 15 as a vaporous azeotropic mixture with water. The organic layer, together with a cyclohexanone-containing mixture yet to be discussed, is fed, through line 17/18, to distillation column 19. Here a light fraction of constituents of a boiling point lower than that of cyclohexanol and a cyclohexanone is distilled off and carried off through line 20. The distillation residue passes through line 21 to a second distillation column 22, where pure cyclohexanone is distilled off and recovered through line 23. The distillation residue is fed, through line 24, to a third distillation column 25. Here pure cyclohexanol is distilled off, which is carried off through line 26. A distillation residue leaves the system through line 27. If desired, the cyclohexanol can be extracted, wholly or partly, through line 28, but in this example it passes through line 26A to alcohol dehydrogenation unit 29, where it is (partly) dehydrogenated to cyclohexanone by a known method, e.g. with a zinc or copper catalyst. The hydrogen obtained is separated off and returned (not drawn) to hydrogenation reactor 5, and the cyclohexanol/cyclohexanone mixture obtained passes through line 30/18 to distillation column 19. The aqueous layer of the product mixture from ester hydrolysis reactor 15 is carried off through line 31. If necessary, the sulphuric acid is concentrated, e.g. by evaporation, and returned to sulphuric acid addition reactor 12. Through line 32 the vaporous mixture of cyclohexene, benzene, cyclohexane and water goes to dehydrogenation reactor 33. Here cyclohexane and cyclohexene are dehydrogenated to benzene at 370°C and atmospheric pressure with 0.6% by weight of platinum on aluminium oxide as catalyst. The degree of conversion of the cyclohexane is 98%, that of the cyclohexene virtually 100%. The conversion efficiency is 98%. Through line 34 the reaction mixture is fed to condenser/separator 35. The non-condensed gas, mainly hydrogen, is returned, through lines 36/2, to hydrogenation reactor 5. The aqueous phase of the condensate is carried off through line 37, and the organic phase is fed, through line 38 to distillation column 39. Here a light fraction consisting mainly of the methyl cyclopentane byproduct with benzene carried along azeotropically, is distilled off and discharged from the system through line 40. The distillation residue consisting mainly of benzene is returned, through lines 41/4, to hydrogenation reactor 5. If desired, the benzene can first be separated by distillation from some heavier impurities.

Figure 2: The first (hydrogenation) and the last (dehydrogenation) section are the same as described in figure 1. In the embodiment of figure 2 the hydration section of figure 2 is replaced by an oxidation section. Therefore only this oxidation section is described here.

Now the organic phase from separator 8 is fed, through line 11, to oxidation reactor 112. Through line 113 an aqueous solution is fed to reactor 112, containing $PdCl_2$ and $CuCl_2$. The pH of this solution is between —2 and +4. The oxidation reaction is carried out at 100°C under autogenic pressure. The molar ratio between cyclohexene and $PdCl_2$ of the supply to 112 is 100:1. The reaction mixture then is fed to separator 115 through line 114, where the catalyst containing aqueous phase is separated from the organic phase. The aqueous catalyst solution is fed to reoxidation reactor 117 through line 116. Through line 118 air is fed to reactor 117. The reoxidation is carried out at 100°C and 1 MPa. The reoxidized catalyst solution is recirculated to oxidation reactor 112 through line 113. The exhausted air is blown off through line 119. The organic phase from separator 115 is fed to distillation unit 121 through line 120. Here a mixture of benzene, cyclohexene and cyclohexane is distilled off.

The bottom product of distillation unit 121 is fed to a second distillation column 125 through line 124. Here pure cyclohexanone is distilled off and is recovered through line 126. The distillation residue of 125 leaves the system through line 127. The benzene-cyclohexene-cyclohexane mixture from 121 is fed to dehydrogenation reactor 33 through line 122. If wanted steam may be supplied to 33 via line 128.

From here the description of figure 2 is the same again as the description of figure 1 (see above).

## Claims

1. Method for the preparation of cyclohexanol and/or cyclohexanone by hydrogenating benzene to cyclohexene, hydrating the cyclohexene to cyclohexanol and/or oxidating it to cyclohexanone, recovering the cyclohexanol and/or cyclohexanone, and returning unconverted benzene and/or cyclohexene to the hydrogenation step, this method being characterized in that the mixture of cyclohexane with benzene and/or cyclohexene, which has also been obtained during the separation of cyclohexanol from the reaction mixture of the hydration step and/or of cyclohexanone from the reaction mixture of the oxidation step, is subjected to a dehydrogenation reaction, and the benzene thus obtained is returned to the hydrogenation step.

2. Method according to claim 1, characterized in that, in the hydrogenation step, a degree of benzene conversion is applied corresponding with 25—75 moles % of cyclohexane in the product mixture.

3. Method according to claims 1 or 2, characterized in that, in the hydrogenation step a ruthenium catalyst is used and a degree of benzene conversion of at least 30% is applied.

4. Method according to claim 3, characterized in that, in the hydrogenation step, a degree of benzene conversion of 40—80% is applied.

5. Method according to one of the claims 1—4, characterized in that, the hydrogen obtained in the dehydrogenation step is returned in the hydrogenation step.

6. Cyclohexanol and/or cyclohexanone obtained by means of the method according to one of the above claims.

## Patentansprüche

1. Verfahren zur Herstellung von Cyclohexanol und/oder Cyclohexanon durch Hydrierung von Benzol zu Cyclohexen, Hydratisierung des Cyclohexens zu Cyclohexanol und/oder Oxydierung des Cyclohexens zu Cyclohexanon, Rückgewinnung des Cyclohexanols und/oder Cyclohexanons und Rückführung des nichtumgewandelten Benzols und/oder Cyclohexens zum Hydrierschritt, welches Verfahren dadurch gekennzeichnet ist, dass das Gemisch von Cyclohexan mit Benzol und/oder Cyclohexen, welches auch während der Trennung der Cyclohexanols vom Reaktionsgemisch des Hydratisierungsschrittes und/oder des Cyclohexanons vom Reaktionsgemisch des Oxydationsschrittes erhalten worden is, einer Dehydrierungsreaktion unterworfen und das so erhaltene Benzol zum Hydrierschritt zurückgeführt wird.

2. Verfahren nach Anspruch 1, dadurch gekennzeichnet, dass im Hydrierschritt ein Benzol-Umwandlungsgrad angewandt wird, der 25—75 Mol-% des Cyclohexans im Produktgemisch enspricht.

3. Verfahren nach den Ansprüchen 1 oder 2, dadurch gekennzeichnet, dass im Hydrierschritt ein Rutheniumkatalysator verwendet wird und ein Benzol-Umwandlungsgrad von mindestens 30% angewandt wird.

4. Verfahren nach Anspruch 3, dadurch gekennzeichnet, dass im Hydrierschritt ein Benzol-Umwandlungsgrad von 40—80% angewandt wird.

5. Verfaren nach einem der Ansprüche 1—4, dadurch gekennzeichnet, dass der im Dehydrierschritt enhaltene Wasserstoff zum Hydrierschritt zurückgeführt wird.

6. Cyclohexanol und/oder Cyclohexanon, erhalten mittels des Verfahrens nach einem der obigen Ansprüche.

**Revendications**

1. Procédé de préparation de cyclohexanol et/ou de cyclohexanone en hydrogénant de benzène en cyclohexène, en hydratant le cyclohexène en cyclohexanol et/ou en l'oxydant en cyclohexanone, en séparant le cyclohexanol et/ou le cyclohexanone, en ramenant le benzène et/ou le cyclohexène non convertis à la phase d'hydrogénation, lequel procédé se caractérise en ce que le mélange de cyclohexane avec du benzène et/ou du cyclohexène, qui a été obtenu lors de la séparation du cyclohexanol du mélange réactionnel de la phase d'hydrogénation et/ou lors de la séparation de la cyclohexanone du mélange réactionnel de la phase d'oxydation, est soumis à une réaction de déshydrogénation, et que le benzène ainsi obtenu est ramené à la phase d'hydrogénation.

2. Procédé selon la revendication 1, caractérisé en ce que, dans la phase d'hydrogénation, on applique un degré de conversion du benzène correspondant avec 25—75 moles % de cyclohexane dans le mélange de produit.

3. Procédé selon l'une des revendications 1 à 2, caractérisé en ce que, dans la phase d'hydrogénation, on utilise un catalyseur de ruthénium et un degré de conversion du benzène de 30% au minimum.

4. Procédé selon la revendication 3, caractérisé en ce que, dans la phase d'hydrogénation, on applique un degré de conversion du benzène de 40—80%.

5. Procédé selon l'une des revendications 1 à 4, caractérisé en ce que l'hydrogène obtenu dans la phase de déshydrogénation est ramené à la phase d'hydrogénation.

6. Cyclohexanol et/ou cyclohexanone obtenus par la mise en oeuvre de procédé selon l'une ou l'autre des revendications ci-dessus.